**Europäisches Patentamt**

**European Patent Office**

**Office européen des brevets**

(19)

(11) Veröffentlichungsnummer: **0 014 834**
**B1**

(12) **EUROPÄISCHE PATENTSCHRIFT**

(45) Veröffentlichungstag der Patentschrift: 03.11.82

(21) Anmeldenummer: 80100152.0

(22) Anmeldetag: 14.01.80

(51) Int. Cl.³: **C 07 C 102/04,**
C 07 C 103/52
//C07D231/50, C07D333/38,
C07F9/38

(54) Verfahren zur Herstellung von Carbonsäureamiden und Peptiden.

(30) Priorität: 18.01.79 DE 2901843

(43) Veröffentlichungstag der Anmeldung:
03.09.80 Patentblatt 80/18

(45) Bekanntmachung des Hinweises auf die Patenterteilung:
03.11.82 Patentblatt 82/44

(84) Benannte Vertragsstaaten:
AT BE CH DE FR GB IT NL SE

(56) Entgegenhaltungen:
FR - A - 2 159 477
FR - A - 2 174 505
FR - A - 2 358 414
GB - A - 714 427
US - A - 1 972 142
US - A - 4 043 991
US - A - 4 128 542

ORGANIC REACTIONS, Band 12, 1962, Seiten
262—278, John Wiley & Sons, Inc. "Alpha-
Acylaminoacyl phosphates" New York, U.S.A.
TETRAHEDRON LETTERS, Nr. 40, 1976, Seite
3583, Pergamon Press, G.B. A. G. JACKSON et
al.: "Activation of carboxylic acids as diphenylphosphinic mixed anhydrides: application to
peptide chemistry"

(73) Patentinhaber: HOECHST AKTIENGESELLSCHAFT
Postfach 80 03 20
D-6230 Frankfurt/Main 80 (DE)

(72) Erfinder: Wissmann, Hans, Dr.
Falkenstrasse 12
D-6232 Bad Soden am Taunus (DE)
Erfinder: Kleiner, Hans-Jerg, Dr.
Altkönigstrasse 11a
D-6242 Kronberg/Taunus (DE)

(56) Entgegenhaltungen:
CHEMICAL ABSTRACTS, Band 53, Nr. 1, 10.
Januar 1959, Spalte 244i Columbus, Ohio, U.S.A.
F. CRAMER et al.: "Chemistry of high-energy
phosphates. V. Peptide synthesis via phosphoric
acid-amino acid anhydrides" & J. AM. CHEM.
SOC. 91, 1562—6 1958
METHODEN DER ORGANISCHEN CHEMIE, Eugen
Müller, (Houben-Weyl) Band XV/2, Synthese von
Peptiden Teil II, 1974, Seiten 226—230 Georg
Thieme Verlag, Stuttgart, DE. "Anhydride mit
Sauren des Phosphors, Anhydride mit Phosphorsäuren, Anhydride mit O-Alkyl- und O-
Arylphosphorsäuren"
METHODEN DER ORGANISCHEN CHEMIE, Eugen
Müller, (Houben-Weyl), Band XV/2, Synthese von
Peptiden Teil II, 1974, Seiten 246—250 Georg
Thieme Verlag, Stuttgart, DE. "Das "Mitin-
Verfahren" und verwandte Methoden"

# Verfahren zur Herstellung von Carbonsäureamiden und Peptiden

Gegenstand der Erfindung ist ein Verfahren zur Herstellung von Carbonsäureamiden und Peptiden, dadurch gekennzeichnet, daß man Verbindungen, die eine freie Aminogruppe enthalten, in Gegenwart von Anhydriden der Alkanphosphonsäuren mit Verbindungen umsetzt, die eine freie Carboxylgruppe besitzen.

Das erfindungsgemäße Verfahren eignet sich sowohl für die Herstellung von Carbonsäureamiden, als auch besonders zur Herstellung von Peptiden.

Als Carbonsäureamide kommen vor allem Amide von aromatischen, aliphatischen und heterocyclischen Carbonsäuren in Betracht, z.B. ggf. durch Alkyl-, Alkoxy-, Nitro-, Dialkylamino-, Acylamino- oder Nitrilgruppen substituierte Benzoesäuren, Naphthoesäuren oder Anthracensäuren weiterhin Phenylalkylcarbonsäuren mit geradkettigen oder verzweigten Alkylresten bis zu 10 C-Atomen, die ggf. die genannten Substituenten am Benzolkern enthalten können, weiterhin N- oder S-haltige Heterocyclen mit 5- und 6-Ring, die ebenfalls die gleichen Substituenten tragen können.

Als Aminokomponenten für die Herstellung der Carbonsäureamide kommen außer Ammoniak auch aliphatische, aromatische oder heterocyclisch substituierte primäre und sekundäre Amine mit den vorstehend beschriebenen Substituenten in Frage.

Zur Herstellung von Aminocarbonsäureamiden oder Peptiden arbeitet man mit solchen Aminocarbonsäurederivaten oder Peptiden, deren Carboxylgruppe geschützt ist und setzt diese mit Aminocarbonsäuren oder Peptiden um, die eine freie Carboxylgruppe besitzen, deren Aminogruppe jedoch geschützt ist. Andere funktionelle Gruppen in den Aminosäuren- oder Peptiden werden zweckmäßig durch die üblichen Peptid-Schutzgruppen geschützt, diese Schutzgruppen können anschließend an das erfindungsgemäße Verfahren in üblicher Weise abgespalten werden.

Bei der Herstellung von Carbonsäureamiden arbeitet man bei Raumtemperatur oder auch bei mäßig erhöhter Temperatur ca. 60°, bei der Herstellung von Aminocarbonsäureamiden oder Peptiden bei Temperaturen von −10°C bis +40°C, vorzugsweise zwischen 0° und Zimmertemperatur.

Zur Wasserabspaltung nach dem erfindungsgemäßen Verfahren geeignet sind die Anhydride der gerad- oder verzweigtkettigen, gegebenenfalls cyclischen Alkanphosphonsäuren mit Kettenlängen von 1 bis 8 Kohlenstoffatomen, bevorzugt bis zu 4 Kohlenstoffatomen.

Als Anhdride der Alkanphosphonsäuren seien beispielsweise genannt: Methan-phosphonsäureanhydrid. Äthan-phosphonsäureanhydrid, n-Propan-phosphonsäureanhydrid, n-Butanphosphonsäureanhydrid.

Die Herstellung der Alkanphosphonsäureanhydride kann in an sich bekannter Weise erfolgen, wie z.B. in Houben-Weyl, Meth. d. Org. Chem. (1963) Bd. XII/1, S. 612 formuliert.

Für die Herstellung von Peptiden arbeitet man zweckmäßig mit besonders reinen Alkanphosphonsäuren-Anhydriden. Derartige Anhydride lassen sich gewinnen durch Umsetzung reiner Alkanphosphonsäuredichloride mit 1 Mol Wasser und anschließender Entfernung des noch im Reaktionsgut verbliebenen Chlorwasserstoffs durch Anlegen eines Vakuums. Bevorzugt können diese Anhydride weiterhin nach dem Verfahren der deutschen Patentanmeldung P 28 11 628.1 hergestellt werden. Hierbei werden reine Alkanphosphonsäuren durch thermische Wasserabspaltung in die Anhydride überführt; eine anschließende Reinigung mit Hilfe einer Vakuumdestillation kann zweckmäßig sein.

Die erfindungsgemäße Umsetzung erfolgt, wenn Peptide synthetisiert werden sollen, zweckmäßigerweise in neutralem oder höchstens schwach alkalischem Medium.

Am einfachsten ist es, eine Pufferung des Mediums durch Zugabe von aliphatischen und cycloaliphatischen tertiären Basen, wie z.B. N-Methylmorpholin, N-Äthylmorpholin, Trialkylaminen mit bis zu 6 C-Atomen pro Alkylrest vorzunehmen. Die erfindungsgemäß verwendeten Alkanphosphonsäureanhydride sind meist gut löslich in Lösungsmitteln wie z.B. Dimethylsulfoxyd, DMF, DMA, Diäthylphosphit, 1-Methyl pyrrolidon, Chloroform und Methylenchlorid.

Zur Herstellung von Oligopeptiden verwendet man als Ausgangsmaterialien eine Aminosäure oder ein Peptid mit einer blockierten Carboxylgruppe und eine Aminosäure oder ein Peptid mit einer blockierten Aminogruppe. Für den Schutz der Carboxylgruppe und der Aminogruppe können alle in der Peptidsynthese üblichen Schutzgruppen verwendet werden. Für den Schutz der Carboxylgruppe werden z.B. Ester geradkettiger und verzweigtkettiger aliphatischer Alkohole, wie z.B. Methanol, Äthanol, tert.-Butanol verwendet. Vgl. Houben-Weyl, Methoden d. Org. Chemie Bd. 15/1 (1974) Synthese von Peptiden, S. 315—350. Auch Ester araliphatischer Alkohole, wie z.B. Benzylalkohol und Biphenylmethylcarbinol können Verwendung finden.

Zum Schutz der Aminogruppen können beispielsweise der Carbobenzoxyrest und der Carbo-tert.-butyloxyrest verwendet werden, Vgl. Houben-Weyl, Methoden d. Org. Chemie 15/1 (1974) S. 47 und 117ff. Als Lösungsmittel können alle in der Peptidsynthese üblichen wasserfreien, inerten Lösungsmittel, wie z.B. Methylenchlorid, Chloroform, Dimethylformamid, Dimethylacetamid, Dioxan, Diäthylphosphit, 1-Methylpyrrolidon und Tetrahydrofuran Verwendung finden.

Die erfindungsgemäßen Alkanphosphonsäureanhydride werden vorzugsweise im Überschuß (2—2,5 Mol Alkanphosphonsäureanhydrid/Mol zu knüpfender Peptidbindung) eingesetzt.

Das erfindungsgemäße Verfahren hat erhebliche Vorteiler:

Bei den dem Synthesereagenzen zugrundeliegenden Säuren ist bisher keine Allergienwirkung beobachtet worden. Die Toxizität ist gering. Das Reagenz selbst, das einfach herzustellen ist, liefert insbesondere bei der Verwendung von destillierten Alkanphosphonsäureanhydriden, nach der Synthese keine schwerlöslichen Nebenprodukte, wie sie z.B. bei der häufig verwendeten Peptidknüpfung unter Verwendung von Dicyclohexyl-carbodiimid auftreten.

Gegenüber den bisher beschriebenen Verfahren der Peptidsynthese unter Verwendung von Aktivierungsmitteln auf der Basis des 3- oder 5-wertigen Phosphors, wie z.B. den Peptidsynthesen nach der Phosphorazomethode (Liebigs Ann. Chem. 580, S. 68 (1953) den Synthesemethoden unter Verwendung von Diäthylchlorphosphit und Tetraäthylpyrophosphit (J. Am. chem. Soc. 74, 5304, 5307 und 3509) (1952) und der Synthesemethode unter Verwendung von Polyphosphorsäureestern (Ber. *91*, (1958) S. 1073—1082 oder J. org. Chem. *26*, 2534 (1961), hat das erfindungsgemäße Verfahren den Vorteil der geringeren Racemisierung bei der üblichen Verwendung von Aminosäurebeziehungsweise Peptidesterhydrochloriden.

So zeigt z.B. Z-Phe-Gly-OEt, nach dem erfindungsgemäßen Verfahren hergestellt, ein $[\alpha]_D$ von −17° (c=2, Athanol). Die Methode unter Verwendung von Diäthylchlorphosphit (J. Am. Chem. Soc. 74, 5307 (1952) liefert ein Peptid mit einem $[\alpha]_D$ von −16° (c=2.0 Äthanol). Das in Beispiel 4 beschriebene Z-Asp(OBu$^t$)-Phe-NH$_2$ zeigt ein $[\alpha]_D$ von −33,8° (c=1, Äthanol) im Vergleich zum Literaturwert von −30,7° (Phosphorazomethode, Hoppe-Seyler's Z-Physiol. Chem. 353, (1972), S. 1250). Die als Racemisierungstest bekannte Synthese von Z-Gly-Phe-Gly-OEt (J. Am. Chem. Soc. *80* (1958) S. 2902) lieferte mit dem erfindungsgemäßen Verfahren die L-Verbindung in einer Ausbeute von 75% d.Th., vgl. Beispiel 10. In Äthanol schwerlösliche Anteile (DL-Peptide) wurden nicht erhalten. Dagegen fallen bei der Synthese des Tripeptids mit Polyphosphorsäureäthylestern nach (J. Org. Chem. 26, 2534 (1961) 32% in Äthanol schwerlösliche DL-Verbindung, und 32% L-Verbindung an.

Ein weiterer Vorteil des erfindungsgemäßen Verfahrens ist die gute thermische Stabilität des Synthesereagenzes. Gegenüber den Polyphosphorsäureestern ist ferner vor allem die Destillierbarkeit der Alkanphosphonsäureanhydride als Vorteil zu vermerken, die den Weg zu definierten Oligomeren öffnet.

Die starke lösungsvermittelnde Wirkung der Alkanphosphonsäureanhydride und ihrer Folgeprodukte im Verlauf der Peptidsynthese, ist ferner, besonders bei der Synthese längerer Peptidketten und schwerlöslicher Peptidsequenzen sehr erwünscht.

Die Verfahrensprodukte werden als Zwischenprodukte oder zur Herstellung von pharmazeutisch werksamen Peptiden verwendet.

Beispiel 1:
Carbobenzoxy-glyzylglyzinäthylester

Zu der Lösung von 10,5 g (0,05 Mol) Carbobenzoxy-glyzin in 100 ml Dimethylformamid gibt man unter Rühren und guter Kühlung nacheinander bei 0° 7,0 g (0,05 Mol) H-Gly-OC$_2$H$_5$ . HCl, 15 ml (0,118 Mol) N-Äthylmorpholin und 36 ml einer 55%igen (W/V) Lösung von n-Propanphosphonsäureanhydrid (0,1 Mol) in Methylenchlorid zu. Man läßt unter Rühren auf Raumtemperatur erwärmen. Nach 16-stündigem Stehen bei Raumtemperatur destilliert man das Lösungsmittel i.V. ab, und nimmt den Rückstand in einem Gemisch aus 200 ml Essigester und 100 ml 5%iger Kaliumbisulfatlösung auf. Die Essigesterlösung wird noch zweimal mit je 100 ml gesättigter Natriumbicarbonatlösung gewaschen, über Natriumsulfat getrocknet, und i.V. abgedampft.

Ausbeute: 11,8 g Z-Dipeptidester mit einem Fp von 81° (80% d.Th.) nach Umkrist, aus Essigester/Petroläther: 82°

Beispiel 2:
Z-Val-Tyr(Bu$^t$)His-OCH$_3$

Zu einer Suspension von 11,5 g H-His-OCH$_3$, HCl in 100 ml Dimethylformamid gibt man nacheinander bei 0° unter Rühren 30 ml N-Äthylmorpholin, 21,5 g Z-Val-Tyr-OH, und 40 ml einer 38%igen Lösung von n-Propanphosphonsäureanhydrid in Methylenchlorid bei Temperaturen bis 10°. Die nach Abklingen der exothermen Reaktion praktisch klare Reaktionslösung läßt man über Nacht bei Raumtemperatur stehen, dann dampft man die Lösungsmittel i.V. bei Raumtemperatur ab, versetzt den Rückstand mit einem Gemisch aus 100 ml gesättigter NaHCO$_3$-Lösung und 200 ml Essigsäureäthylester. Das Rohprodukt wird unter Schütteln in die Essigesterphase überführt, diese wird mit wenig Wasser gewaschen, über Natriumsulfat getrocknet, und i.V. zur Trockne gebracht. Der im Rückstand verbliebene Z-Tripeptidester erstarrt beim digerieren mit Diäthyläther.

Ausbeute: 20 g, vom Fp. 188—190° $[\alpha]_D$=−8,2° (c=1, DMF) Aus der Mutterlauge können durch Abdampfen des Lösungsmittels und Umfällen aus Essigsäureäthylester/Diäthyläther weitere 3,5 g des Peptids gewonnen werden. Gesamtausbeute: 76% d.Th.

Beispiel 3:
Z-Pro-Ala-Lys-(Boc)-Phe-NH$_2$

19,1 g H-Lys(Boc)-PHe-NH$_2$.HCl (0,044 Mol)

löst man in 100 ml Dimethylformamid, gibt man bei 0° unter Rühren 26 ml N-Äthylmorpholin, 16,0 g (0,005 Mol) Z-Pro-Ala-OH und 22 g n-Propanphosphonsäureanhydrid als 38%ige (W/V) Lösung in Methylenchlorid zu. Man läßt 48 Stunden bei Raumtemperatur stehen, dann bringt man die Reaktionsmischung im Vakuum bei Raumtemperatur zur Trockene, digeriert den Rückstand mit 100 ml 2n-Sodalösung, 100 ml 10%iger wässriger Zitronensäurelösung und 100 ml dest. Wasser und trocknet i.V. über Phosphorpentoxyd.

Ausbeute: 30,1 g=87% d.Th. $[\alpha]_D=-27,0°$ (=1, DMF); Fp. 163°

Beispiel 4:
Z-Asp(OBu$^t$)-Phe-NH$_2$

Man löst 1,6 g (0,01 Mol) H-Phe-NH$_2$ zusammen mit 28 ml (0,22 Mol) N-Äthylmorpholin bei 0° in 20 ml Dimethylformamid. Dieser Lösung setzt man unter Rühren und Kühlung 3,23 g (0,01 Mol) Z-Asp(OBu$^t$)OH und 11,4 ml 38%ige n-Propanphosphonsäureanhydridlösung in Methylenchlorid zu. Die so hergestellte Reaktionslösung läßt man über Nacht bei Raumtemperatur stehen. Nach Abdestillieren des Lösungsmittels und Zusatz von Wasser extrahiert man mit Essigester, wäscht mit Wasser, wässriger Natriumbicarbonatlösung, 5% wässriger KHSO$_4$-Lösung, engt die über Natriumsulfat getrocknete Essigesterlösung und fällt das Endprodukt mit Diäthyläther aus.

Ausbeute: 4,0 g (85% d.Th.) Fp.: 162° $[\alpha]_D=-33,1°$ (c=1), CH$_3$OH)

Beispiel 5:
Boc-Met-Gly-OEt

Zu 115 ml Dimethylformamid werden unter Rühren und Feuchtigkeitsausschluß bei 0° nacheinander 24,8 g Boc-Met-OH 14,0 g H-Gly-OEt . HCl, 60 ml N-Äthylmorpholin und 67 ml einer 67%igen Methylenchloridlösung von n-Propanphosphonsäureanhydrid gegeben. Man rührt bei Raumtemperatur noch 20 Stunden nach, destilliert die Lösungsmittel i.V. bei Raumtemperatur ab und versetzt den Rückstand mit Wasser. Den ausgefallenen Peptidester nimmt man in Essigester auf und wäscht die Essigesterlösung nacheinander mit gesättigter wässr. NaHCO$_3$-Lösung, 5%iger KHSO$_4$-Lösung und Wasser. Aus der über Natriumsulfat getrockneten Essigesterlösung kristallisiert nach weitgehendem Abdampfen des Lösungsmittels i.V. bei Raumtemperatur der Acylpeptidester aus. Es wird mit wenig abs. Diäthyläther gewaschen und abgesaugt.

Ausbeute nach Trocknen i.V. über P$_2$O$_5$: 29,5 g=93% d.Th. Fp.: 53°, $[\alpha]_D=-15,4°$ (c=1, DMF)

Beispiel 6:
Boc-Tyr(Etoc)-Met-Gly-OC$_2$H$_5$

Man löst 23 g H-Met-Gly-OC$_2$H$_5$ . HCl in 200 ml Dimethylformamid und gibt unter starker Kühlung bei 0° 58 ml N-Äthylmorpholin, 31.5 g Boc-Tyr(Etoc)-OH, und 44 g n-Propanphosphonsäureanhydrid, gelöst in 60 ml Methylenchlorid, zu, Man läßt die Lösung 20 Stunden bei Raumtemperatur stehen, destilliert dann die Lösungsmittel im Vakuum bei Raumtemperatur ab, und arbeitet das Reaktionsgemisch wie in Beispiel 5 auf.

Ausbeute: 40,7 g (84% d.Th.) Fp.: 137,5° $[\alpha]_D=-11,7°$ (c=1, DMF)

Beispiel 7:
Z-Gly-Thr(Bu$^t$)Phe-OCH$_3$

In 120 ml Dimethylsulfoxyd (p. A. Merck) löst man nacheinander 18,6 g (0,05 Mol) H-Thr(Bu$^t$)-Phe-OCH$_3$ . HCl, 30 ml (0,238 Mol) N-Äthylmorpholin, und 10,4 g (0,05 Mol) Z-Gly-OH und gibt dann unter Eiskühlung und Feuchtigkeitsausschluß 16,3 g Methanphosphonsäureanhydrid portionsweise unter Rühren zu. Das Methanphosphonsäureanhydrid geht langsam in Lösung. Man rührt noch 24 Std. bei Raumtemperatur nach, und gießt dann die Reaktionslösung in 500 ml gesättigte Natriumbicarbonatlösung ein. Das Reaktionsprodukt fällt dabei aus. Man dekantiert von der überstehenden Lösung, und nimmt die Fällung in Essigsäureäthylester auf. Die Essigesterlösung wäscht man mit Wasser, trocknet über Magnesiumsulfat, engt i.V. weitgehend ein, und fällt das Endprodukt mit Petroläther. Es kristallisiert über Nacht bei +4°.

Beispiel 8:
Z-Phe-cyclohexylamid

Man löst 30 g (0,01 Mol) Z-Phe-OH, 1,0 g (0,01 Mol; 1,2 ml) Cyclohexylamin, und 5 ml N-Äthylmorpholin in 30 ml DMF, und gibt dann unter Eiskühlung 8,8 g einer 50-gewichtsprozentigen n-Propanphosphonsäureanhydridlösung in Methylchlorid zu (0,0435 Mol). Nach Abklingen der exothermen Reaktion (Temperaturanstieg bis+10°) läßt man die Lösung unter Rühren auf Raumtemperatur erwärmen, und arbeitet dann das Reaktionsgemisch wie unter Beispiel 1 beschrieben auf.

Ausbeute: 3,1 g (81% d.Th.) $[\alpha]_D=-2,8°$ (c=1, DMF) Fp.: 167°

Beispiel 9:
Z-Ala-Tyr-Gly-Leu-Arg-Pro-Gly-NH$_2$

Eine Mischung von 4,99 g (0,01 Mol Z-Ala-Tyr-Gly-OH, 5,13 g (0,01 Mol) H-Leu-Arg-Pro-Gly-NH$_2$ . 2 HCl, 20 ml Dimethylformamid, und 10 ml (0,078 Mol) N-Äthylmorpholin wird unter Rühren und Feuchtigkeitsausschluß auf −10°C gekühlt. Anschließend tropft man unter weiterem Rühren 6,6 g (0,022 Mol) n-Hexanphosphonsäureanhydrid als 50%ige Lösung in Methylenchlorid (p.A., dest, über $P_2O_5$) zu. Die Temperatur soll während des Zutropfens+10°C nicht übersteigen. Man rührt die entstandene Lösung noch 3 Stunden nach, und läßt über Nacht stehen. Anschliessend engt man i.HV. bei Raumtemperatur auf ca. 30 ml ein, versetzt mit 100 ml gesättigter Natriumbicarbonatlösung und extrahiert die Mischung dreimal mit insgesamt 400 ml Essigester. Die Essigesterlösung extrahiert man noch je zweimal mit 50 ml gesättigter Natriumbicarbonatlösung und Wasser, trocknet über Natriumsulfat und isoliert das Produkt durch Abdampfen der Essigesterlösung i.V., verreiben des Rückstandes mit abs. Diäthyläther.

Ausbeute: 7,46 g (81% d.Th.) Fp.: 130° (Z) $[\alpha]_D$=—29,2° (c=1, DMF)

Herstellung des n-hexanphosphonsäureanhydrids:

200 g n-Hexanphosphonsäure werden in einem Glaskolben mit einer aufgesetzten nicht verspiegelten 12 cm-Vigreux-Kolonne und einem angeschlossenen Luftkühler mit Vorlage erhitzt auf max. 340°C bei einem Druck von ca. 0,3 Torr. Während 10 Std. destilliert bei einer übergangstemperatur von 225° bis 250°C Hexanphosphonsäureanhydrid ab. In einer der Apparatur nachgeschalteten Kühlfalle kondensieren ca. 22 g Wasser. Man erhält 170 g des Anhydrides. Das Produkt wird vor der Umsetzung nochmals fraktioniert. Es ist klar löslich in Methylenchlorid, Chlorform Dimethylformamid, Dimethylsulfoxyd, Petrolätherv. Kp 40—80°, Äther, Toluol, Dioxan, Cyclohexan, Tetrahydrofuran.

Beispiel 10:
Z-Gly-Phe-Gly-OEt

Man suspendiert 1,4 g (0,01 Mol) H-Gly-OEt. HCl und 3,56 g (0,01 Mol) Z-Gly-Phe-OH unter Rühren in 30 ml Dimethylformamid und gibt nach Abkühlen auf —5° unter Rühren und Feuchtigkeitsausschluß 5,6 ml (0,044 Mol) N-Äthylmorpholin zu. Dann tropft man unter Rühren und Kühlung 8,8 g einer 50%igen Methylenchloridlösung von n-Propanphosphonsäureanhydrid (0,021 Mol) zu. Die Temperatur des Reaktionsgemisches soll dabei +10° nicht überschreiten. Anschließend rührt man noch 1 Stunde nach, und läßt 48 Stunden bei Raumtemperatur stehen. Man destilliert die Lösungsmittel i.HV. ab, und setzt 50 ml Eiswasser zu. Das ausgefallene Endprodukt wird nach Kristallisation abgesaugt, mit Natriumbicarbonatlösung, Zitronensäurelösung (5%ig) und Wasser gewaschen, und über $P_2O_5$ im Vakuum getrocknet.

Ausbeute: 4,30 g (98% d.Th.) $[\alpha]_D$=—11,9° (c—2, Äthanol) Fp.: 119°

Beispiel 11:
1-Phenyl-2,3-dimethyl-4-(4-oxyphenyl-propionylaminopyrazolon (5)

Zu der unter Feuchtigkeitsausschluß gerührten Lösung von 16,6 g (0,1 Mol) p-Oxyphenylpropionsäure 21 (0,1 g Mol) 1-Phenyl-2,3-dimethyl-4-aminopyrrolidon (5), und 68 ml N-Äthylmorpholin tropft man 88 g einer 50 %igen Lösung von Propanphosphonsäureanhydrid in Methylenchlorid. Die Temperatur wird hierbei, gegebenenfalls durch Drosseln des Zustroms an Propanphosphonsäureanhydrid, unterhalb von +40°C gehalten. Man rührt noch 6 Stunden nach, und läßt dann bei Raumtemperatur 24 Std. stehen. Dann verdünnt man die Reaktionslösung mit 800 ml Methylenchlorid, wäscht die Lösung je dreimal mit 50 ml 5%iger Kaliumbisulfatlösung, gesättigter Natriumbicarbonatlösung, und Wasser.

Man trocknet über Magnesiumsulfat. Das Produkt kristallisiert beim Einengen der getrockneten Methylenchloridlösung im Vakuum bei Raumtemperatur. Eine geringere Restfraktion kann noch aus dem mit Petroläther gefällten Filtrat erhalten werden.

Gesamtausbeute: 25,0 g=70% d.Th. vom Fp.: 218°

Beispiel 12:
Benzoesäure-ß-phenyläthylamid

Zu der Lösung von 2,4 g ß-Phenyläthylamin, 2,2 g Benzoesäure und 14 ml N-Äthylmorpholin in 25 ml Methylenchlorid gibt man unter Rühren und Feuchtigkeitsausschluß in 3 Portionen 17,6 g einer 50%igen Lösung von n-Propanphosphonsäureanhydrid in Methylenchlorid. Die Temperatur steigt dabei bis etwa +40°C. Man läßt unter Rühren auf Raumtemperatur abkühlen und arbeitet dann nach 24-stündigem Stehen auf, indem man 350 ml Methylenchlorid zugibt, und die Lösung dreimal mit je 50 ml Wasser, gesättigter Natriumcarbonatlösung und Kaliumbisulfatlösung extrahiert. Aus der über Magnesiumsulfat getrockneten Methylenchloridlösung kristallisiert das Rohprodukt. Es wird zusammen mit wenig Petroläther (Kp 40—60°) abgesaugt.

Ausbeute; 3.8 g,=84% d.Th. Fp.: 111—112°, nach Umkristallisieren aus Äthanol: 116° (Blättchen)

Beispiel 13:
Thiophen-2-carbonsäure-ß-phenyläthylamid

Analog zu Beispiel 12 erhält man aus 2,6 g Thiophen-2-carbonsäure, 2,4 g ß-Phenyläthylamin, 14 ml N-Äthylmorpholin gelöst in 15 ml Methylenchlorid, und 17,6 g einer 50%igen Lösung von Propanphosphonsäureanhydrid in Methylenchlorid Thiophen-2-carbonsäure-2-phenyläthylamid.

Ausbeute: 3,5 g,=76% d.Th. Fp: 108—109°, nach Umkristallisieren aus Äthanol: 111°

**Beispiel 14:**

Benzoesäure-4-nitroanilid

Zu der Mischung von 2,8 g 4-Nitroanilin, 15,5 ml Triäthylamin 2,2 g Benzoesäure und 15 ml Methylenchlorid gibt man unter Rühren und Kühlung in 3 Portionen insgesamt 17,6 g einer 50%igen Lösung von n-Propanphosphonsäureanhydrid in Methylenchlorid zu. Man ruhrt noch eine Stunde bei Raumtemperatur nach, in dieser Zeit klärt sich die Mischung. Nach 32 Stunden Stehen bei Raumtemperatur arbeitet man wie unter Beispiel 1 beschrieben, auf.

Ausbeute nach Umkristallisation aus Äthanol; 3,38 g=70% d.Th. F. 198°

**Patentanspruch:**

Verfahren zur Herstellung von Carbonsäureamiden und Peptiden, dadurch gekennzeichnet, daß man Verbindungen, die eine freie Aminogruppe enthalten, in Gegenwart von Anhydriden der Alkanphosphonsäuren mit Verbindungen umsetzt, die eine freie Carboxylgruppe besitzen.

**Revendication**

Procédé de préparation d'amides d'acides carboxyliques et de peptides, caractérisé en ce que on fait réagir des composés qui contiennent un groupe amino libre, en présence d'anhydrides d'acides alcane phosphoniques, avec des composés qui possèdent un groupe carboxyle libre.

**Claim**

Process for the preparation of carboxylic acid amides and peptides, which comprises reacting compounds which contain a free amino group in the presence of anhydrides of alkanephosphonic acids with compounds which contain a free carboxyl group.